# EUROPEAN PATENT SPECIFICATION

(11) **EP 3 763 340 B1**
(45) Date of publication and mention of the grant of the patent: **14.08.2024**
(21) Application number: 20181740.0
(22) Date of filing: 24.03.2017
(51) Int. Cl.: A61F 2/915, A61F 2/88

(54) **VASCULAR FLOW DIVERSION**
GEFÄSSSTRÖMUNGSUMLENKUNG
DÉRIVATION DE FLUX VASCULAIRE

(30) Priority: 24.03.2016 US 201662313055 P
(43) Date of publication of application: 13.01.2021
(62) Divisional of application: 17717280.6
(73) Proprietor: Covidien LP, Mansfield, MA 02048 (US)
(72) Inventor: CHOUBEY, Animesh, Chino, CA 91710 (US); NAGESWARAN, Ashok, Irvine, CA 92606 (US)
(74) Representative: Gray, James

(56) References cited:
- WO-A1-2010/124286
- WO-A1-2012/047308
- WO-A1-2014/153162
- WO-A2-2006/036912
- US-A1- 2004 267 353
- US-A1- 2011 093 059

## Description

### CROSS-REFERENCE TO RELATED APPLICATIONS

The present application claims the benefit of and priority to U.S. Provisional Patent Application No. 62/313,055, filed March 24, 2016.

### TECHNICAL FIELD

The subject technology relates generally to methods and devices for diverting blood flow in a blood vessel, and particularly to inhibiting blood flow into an aneurysm. Some embodiments of the subject technology relate to flow-diverting devices including a plurality of interconnected struts.

### BACKGROUND

Aneurysms are an abnormal bulging or ballooning of a blood vessel that can result from the vessel wall being weakened by disease, injury, or a congenital abnormality. Aneurysms have thin, weak walls and have a tendency to rupture, which can lead to stroke, death, disability, etc. One method of treating aneurysms includes inserting a flow-diverting stent or braid into a parent vessel that includes the aneurysm to be treated. Such stents or braids can be inserted into a vessel in a collapsed state, positioned next to the neck of the aneurysm, and expanded into apposition with the vessel wall. If the stent or braid has a sufficiently low porosity, it can function to block the flow of blood through the device and into the aneurysm to induce embolization of the aneurysm.

However, some aneurysms-and especially cerebral aneurysms-are located in small and tortuous portions of the vasculature. Current designs for flow-diverting stents or braids have difficulty achieving a snug fit across the neck of the aneurysm if the parent vessel is curved, twisted, or forked. For example, current designs generally suffer from crimping or kinking when positioned in such tortuous vessels. This can make it more difficult to position a flow-diverting device and can cause the device to have an inadequate porosity as the device is expanded within the vessel. Also, current designs often undesirably block blood flow to branching or secondary vessels that are close to the aneurysm. Accordingly, there exists a need for improved flow-diverting devices for treating aneurysms. WO2010/124286 discloses a self-expanding flexible or balloon expandable flexible device includes a helical strut member helically wound about an axis of the stent. WO2012/047308 discloses a stent including a first section and a second section. US 2011/093059 discloses a hybrid stent design using half-slot circumferential sets of strut members with short (< 1.5 mm) slot length that has minimal fish scaling and improved stent retention and flexibility.

### SUMMARY

Expandable devices can be delivered into vascular system to divert flow. According to some embodiments, expandable devices are provided for treating aneurysms by diverting flow. A flow-diverting expandable device can comprise a plurality of struts and/or bridges and configured to be implanted in a blood vessel. The expandable device can be expandable to an expanded state at an aneurysm. The expandable device can have at least a section for spanning the neck of the aneurysm and a plurality of pores or openings located between the struts/bridges. The expandable device can have a sidewall and a plurality of pores/openings in the sidewall that are sized to inhibit flow of blood through the sidewall into an aneurysm to a degree sufficient to lead to thrombosis and healing of the aneurysm when the expandable device is positioned in a blood vessel and adjacent to the aneurysm. The subject technology is illustrated, for example, according to various aspects described below.

Further, some embodiments can provide a delivery system for treating an aneurysm. The system can comprise a microcatheter configured to be implanted into a blood vessel, a core member, extending within the microcatheter, having a distal segment, and the device extending along the core member distal segment.

The invention provides an expandable device comprising: a plurality of connector sections, each of the connector sections extending circumferentially about the expandable device and comprising a plurality of connector struts, the connector struts including first connector struts and second connector struts, wherein (a) the first connector struts are generally linear, (b) every other connector strut is a second connector strut, (c) each of the second connector struts comprises a curved section near each end and a straight midsection between the two curved sections, the curved sections of each second connector strut curving in opposite directions, and (d) each of the first connector struts is coupled to one of the second connector struts at an apex; anda plurality of bridge sections, each of the bridge sections attached to and extending between two of the connector sections and comprising a plurality of parallel, non-branching, helical bridge members, wherein at least some of the bridge members directly connect to one of the apices, wherein, for each pair of first connector struts and second connector struts connected by one of the apices, at least the straight midsection of the second connector strut is parallel to at least a portion of the first connector strut.

It is to be understood that both the foregoing general description and the following detailed description are exemplary and explanatory and are intended to provide further explanation of the subject technology.

### BRIEF DESCRIPTION OF THE DRAWINGS

The accompanying drawings, which are included to provide further understanding of the subject technology and are incorporated in and constitute a part of this description, illustrate aspects of the subject technology and, together with the specification, serve to explain principles of the subject technology.
FIG. 1A shows a plan view of an expandable device with a strut pattern, according to some embodiments of the subject technology.
FIG. 1B shows an enlarged plan view of a portion of the expandable device of FIG. 1A, according to some embodiments of the subject technology.
FIG. 2A shows a perspective view of struts, according to some embodiments of the subject technology.
FIG. 2B shows a cross-sectional view of a strut, according to some embodiments of the subject technology.
FIG. 3A shows a plan view of an expandable device with a strut pattern, according to some embodiments of the subject technology.
FIG. 3B shows an enlarged plan view of a portion of the expandable device of FIG. 3A, according to some embodiments of the subject technology.
FIG. 4A shows a plan view of an expandable device with a strut pattern, according to some embodiments of the subject technology.
FIG. 4B shows an enlarged plan view of a portion of the expandable device of FIG 4A, according to some embodiments of the subject technology.
FIG. 5A shows a plan view of an expandable device with a strut pattern, according to some embodiments of the subject technology.
FIG. 5B shows an enlarged plan view of a portion of the expandable device of FIG. 5A, according to some embodiments of the subject technology.
FIG. 6A shows a plan view of an expandable device with a strut pattern, according to some embodiments of the subject technology.
FIG. 6B shows an enlarged plan view of a portion of the expandable device of FIG. 6A, according to some embodiments of the subject technology.
FIG. 7A shows a plan view of an expandable device with a strut pattern, according to some embodiments of the subject technology.
FIG. 7B shows an enlarged plan view of a portion of the expandable device of FIG. 7A, according to some embodiments of the subject technology.
FIG. 8A shows a plan view of an expandable device with a strut pattern, according to some embodiments of the subject technology.
FIG. 8B shows an enlarged plan view of a portion of the expandable device of FIG. 8A, according to some embodiments of the subject technology.
FIG. 9A shows a plan view of an expandable device with a strut pattern, according to some embodiments of the subject technology.
FIG. 9B shows an enlarged plan view of a portion of the expandable device of FIG. 9A, according to some embodiments of the subject technology.
FIG. 10A shows a plan view of an expandable device with a strut pattern, according to some embodiments of the subject technology.
FIG. 10B shows an enlarged plan view of a portion of the expandable device of FIG. 10A, according to some embodiments of the subject technology.
FIG. 11A shows a plan view of an expandable device with a strut pattern, according to some embodiments of the subject technology.
FIG. 11B shows an enlarged plan view of a portion of the expandable device of FIG. 11A, according to some embodiments of the subject technology.
FIG. 12A shows a plan view of an expandable device with a strut pattern, according to some embodiments of the subject technology.
FIG. 12B shows an enlarged plan view of a portion of the expandable device of FIG. 12A, according to some embodiments of the subject technology.
FIG. 13A shows a plan view of an expandable device with a strut pattern, according to some embodiments of the subject technology.
FIG. 13B shows an enlarged plan view of a portion of the expandable device of FIG. 13A, according to some embodiments of the subject technology.
FIG. 14A shows a plan view of an expandable device with a strut pattern, according to some embodiments of the subject technology.
FIG. 14B shows an enlarged plan view of a portion of the expandable device of FIG. 14A, according to some embodiments of the subject technology.
FIGS. 15A to 15D shows a side view of an expandable device in various curved states of different curvatures, according to some embodiments of the subject technology.

### DETAILED DESCRIPTION

In the following detailed description, specific details are set forth to provide an understanding of the subject technology. However, the subject technology may be practiced without some of these specific details. In some instances, well-known structures and techniques have not been shown in detail so as not to obscure the subject technology.

An expandable device comprising a thin film forming a mesh can be used to treat an aneurysm. The expandable device can impede blood flow along an aneurysmal flow path between the prevailing direction of arterial flow and the interior of the aneurysm via, e.g., high pore density, small pore size and/or high material coverage across the aneurysmal flow path, and facilitate endothelial growth across the neck of the aneurysm or otherwise across the aneurysmal flow path. The expandable device can comprise a single component, low profile, high pore density flow diverter of a single material and/or of monolithic construction. The expandable device can facilitate accurate placement by requiring less foreshortening as compared to other commercially available devices, including braided devices. The expandable device can have a thickness that is small enough to enable placement in smaller blood vessels, thereby opening new areas of treatment for flow diversion.

According to some embodiments, an expandable device, such as a stent, can have a flow diverting section or other portion of the device that provides embolic properties so as to interfere with blood flow in (or into) the body space (e.g., an aneurysm) in (or across) which the device is deployed. The sidewall material coverage, porosity, and/or pore size of one or more sections of the device can be selected to interfere with blood flow to a degree sufficient to lead to thrombosis of the aneurysm or other body space.

According to some embodiments, the expandable device can be configured to interfere with blood flow to generally reduce the exchange of blood between the parent vessel and an aneurysm, which can induce thrombosis of the aneurysm. A device (or a device component, such as a sidewall of a stent or a section of such a sidewall) that interferes with blood flow can be said to have a "flow diverting" property.

According to some embodiments, a porosity of the expandable device is equal to a ratio of an open surf ace area of the expandable device to a total surface area of the expandable device. The expandable device may comprise a plurality of struts, which form pores or cells as open areas between the struts.

The device can exhibit a porosity configured to reduce haemodynamic flow into and/or induce thrombosis within an aneurysm. The device can simultaneously allow perfusion to an adjacent branch vessel whose ostium is crossed by a portion of the device. The device can exhibit a high degree of flexibility due to the materials used, the density (i.e., the porosity) of the struts, and the arrangement of struts.

The device can be self-expanding to a relaxed state or an expanded state. As used herein, the relaxed state is one to which the expandable device will self-expand in the absence of any containment or external forces. As used herein, expanded state is one to which the expandable device is capable of self-expanding, ignoring any containment, such by as a blood vessel. For example and simplicity of measurement, this expanded state can be one to which the expandable device will self-expand within a straight, non-tapering cylindrical tube with an inside diameter that is slightly smaller than the maximum diameter of the expandable device in the relaxed state.

The struts and bridge configuration of the expandable device may be formed, for example, by laser cutting a pre-formed tube or sheet, by interconnecting components (e.g., by laser welding), by vapor deposition techniques, or combinations thereof. A more detailed description of methods by which an expandable device may be formed is provided further herein.

According to some embodiments, the expandable device may include a plurality of individual struts and individual cells, as well as a first longitudinal edge and a second longitudinal edge. The first longitudinal edge and the second longitudinal edge may be connected to each other to form a substantially cylindrical shape or a circumferentially continuous cylindrical shape by welding, soldering, or otherwise joining the struts or edges.

According to some embodiments in which the device is not a circumferentially continuous cylinder, the first edge and second edge may be formed, for example, by cutting a preformed, etched or laser-cut tube longitudinally along the length of the tube. Regardless of the manner of forming, the expandable device may be rolled or curled such that the first and second longitudinal edges overlap one another when the expandable device is in a compressed state and/or an expanded state. Upon release from a constraint (e.g. upon exiting a catheter), the expandable device (when configured to be self-expanding) may spring open and attempt to assume an expanded state.

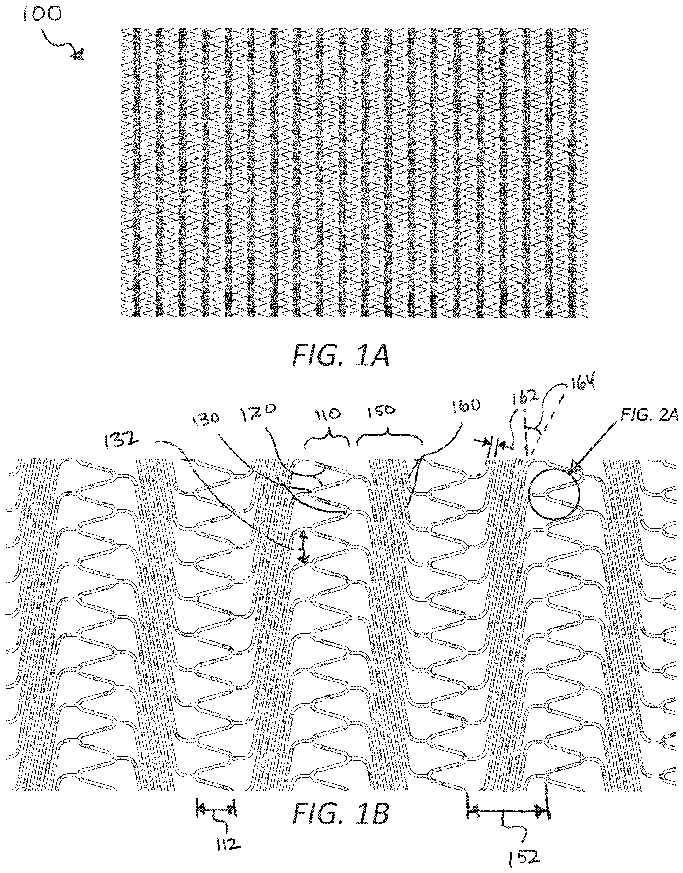

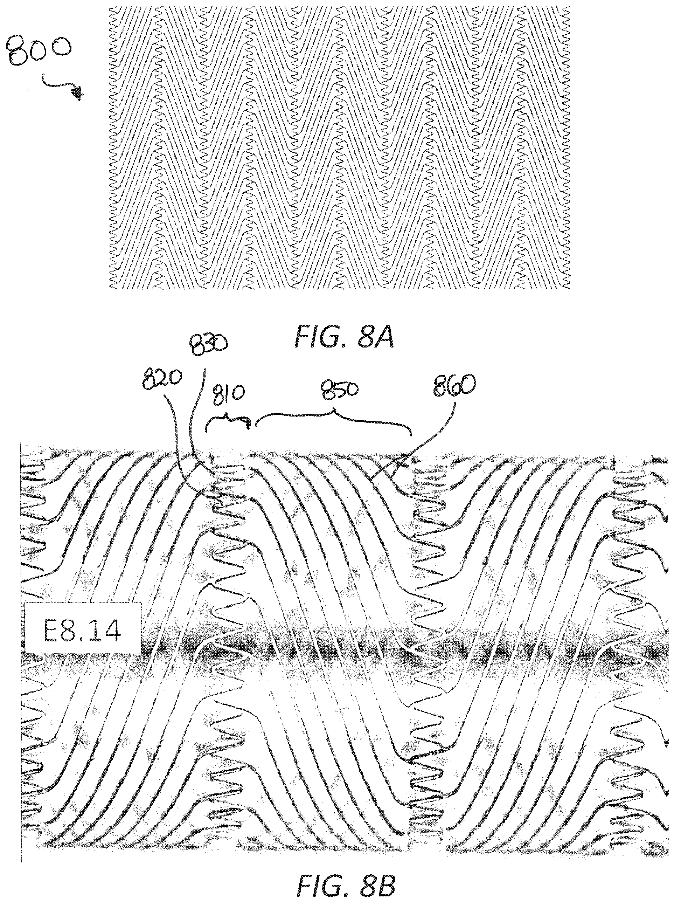

While the views provided in several of the figures (e.g., Figs. 1A, 1B, 3A to 8A, 9A, and 10A to 14B) show expandable devices laid flat for ease of explanation and understanding, it will be understood that the devices can possess a tubular shape (e.g., Figs. 8B, 9B and 15A to 15D), and the laid-flat drawings presented herein depict the configuration of a sidewall of the tube. While in the tubular shape, the expandable devices can have open ends of a lumen extending through the expandable device.

Many embodiments of the subject technology are directed to expandable, flow-diverting mesh devices formed of a non-braided, thin-film mesh structure that includes a plurality of helical bridge struts (described in greater detail below). The mesh devices of the subject technology provide several advantages over conventional, braided flow-diverting devices, especially braided devices. For example, because the mesh devices disclosed herein are non-braided, they foreshorten significantly less than braided devices and thus may be more accurately deployed and positioned within the parent vessel. Moreover, many of the mesh devices disclosed herein are formed of a monolithic piece of metal and thus may have a very small wall thickness (e.g., about 15-20 microns), thereby enabling placement in smaller blood vessels and allowing new anatomical areas of treatment for flow diversion. Finally, because of the density, shape and arrangement of struts, the mesh devices of the subject technology are more flexible than conventional stents and may be positioned around tight corners or bends without kinking.

According to some embodiments, for example as shown in FIGS. 1A and 1B, an expandable device 100 can comprise a plurality of connector struts 120 within a plurality of connector sections 110 and a plurality of bridge members 160 within a plurality of bridge sections 150. Some or all of the bridge sections 150 can be disposed longitudinally between a pair of connector sections 110. Some or all of the connector sections 110 and the bridge sections 150 can extend along some or all of a circumference of the expandable device 100 when the expandable device 100 forms a tubular shape. Some or all of the connector sections 110 can be connected to bridge sections 150 on opposing longitudinal sides of the connector section 110. Some or all of the bridge sections 150 can be connected to connector sections 110 on opposing longitudinal sides of the bridge section 150.

According to some embodiments, for example as shown in FIG. 1B, the connector struts 120 of the connector section 110 can be connected to each other within the connector section 110. As depicted, the connector struts 120 can be arranged in a "zigzag" pattern and the connector section 110 formed thereby can be in the form of a circumferential band, or a V-strut band. An end of one connector strut 120 can be connected to an end of another connector strut 120. One or more connector struts 120 can be connected at an apex 130. Some or all of the apices 130 can be formed at longitudinal ends of the connector section 110, such that each of the apices 130 faces an adjoining bridge section 150. Each connector section 110 can have 28-108 connector struts 120.

According to some embodiments, for example as shown in FIG. 1B, the bridge members 160 of the bridge section 150 can be connected to connector struts 120 of adjacent connector sections 110. Each of the bridge members 160 can connect to a connector strut 120 (e.g., at an apex 130) of one connector section 110 with one end of the bridge member 160 and to a connector strut 120 (e.g., at an apex 130) of another connector section 110 with an opposite end of the bridge member 160. Between the ends of the bridge member 160, the bridge member 160 can be non-branching. Between the ends of the bridge member 160, the bridge member 160 can be unconnected to any other bridge member 160. Each bridge section 150 can have, e.g., 28-108 bridge members 160. Each bridge member 160 can span a circumferential distance of the expandable device 100 while the expandable device 100 is in a tubular shape. For example, each bridge member 160 can span 30° to 180° about the longitudinal axis, for example 120°. By further example, each bridge member 160 can span a distance of 3 to 54 apices 130 between terminal ends of the bridge member 160. At least a portion of a bridge member 160 can be parallel to some or all of the other bridge members 160 of the same bridge section 150 when the expandable device 100 is represented in a laid-flat view such as in Figs. 1A to 1B, etc. At least a portion of a bridge member 160 in a helical shape can be parallel to some or all of the other bridge members 160 in a helical shape of the same bridge section 150 when the expandable device 100 is considered in its tubular form. As used herein, two helical shapes are considered "parallel" if they wind about the same axis, at the same distance (i.e., radius) from the axis, with the same pitch angle or helix angle with respect to the axis, and in the same rotational direction (dextrorotatory or levorotatory) with respect to the axis.

According to some embodiments, a helical winding direction of the bridge members 160 of one bridge section 150 can be different than a helical winding direction of the bridge members 160 of a different bridge section 150. For example, the helical winding direction of some bridge members 160 of one bridge section 150 can be dextrorotatory and the helical winding direction of the bridge members 160 of a different bridge section 150 can be levorotatory. The helical winding direction within any given bridge section 150 can be different than the helical winding direction of any adjacent bridge section 150. For example, alternating bridge sections 150 along a longitudinal length of the expandable device 100 can have alternating helical winding directions relative to each other. When the expandable device 100 is extended longitudinally, the bridge members 160 of the bridge sections 150 can straighten relative to the longitudinal axis, causing the connector sections 110 to rotate about the axis in different directions. This allows the extreme ends of the expandable device 100 to rotate relative to each other less than they would if the bridge members 160 of every bridge section 150 were wound in the same helical direction, or not at all.

According to some embodiments, a bridge gap 162 is a distance between a pair of adjacent bridge members 160. The bridge gap 162 can be measured across parallel portions of pairs of adjacent bridge members 160. The bridge gap 162 can be the same (e.g., uniform) or different among different pairs of bridge members 160 within a single bridge section 150. The bridge gap 162 can be the same/uniform or different among different bridge sections 150 of a single device 100. The bridge gap 162 can be 1 to 250 µm, for example greater than 100 µm.

According to some embodiments, the bridge members 160 form a pitch angle 164 with respect to a line that is orthogonal to the longitudinal axis of the expandable device 100. The pitch angle 164 can be the same/uniform or different for different bridge members 160 within a single bridge section 150. The pitch angle 164 can be the same/uniform or different among different bridge sections 150 of a single device 100. The pitch angle 164 can be 10° to 60°, for example 19°.

According to some embodiments, an apex gap 132 is a distance between a pair of adjacent apices 130 on a same longitudinal side of a connector section 110. The apex gap 132 can be measured as orthogonal to a longitudinal axis of the expandable device 100. The apex gap 132 can be the same/uniform or different among different pairs of apices 130 within a single connector section 110. The apex gap 132 can be the same/uniform or different among different connector sections 110 of a single device 100. The apex gap 132 can be 10 to 450 µm, for example 300 µm.

According to some embodiments, a connector section length 112 is a longitudinal distance between opposing longitudinal sides of a connector section 110 (e.g., between a pair of bridge sections 150). The connector section length 112 can be measured as parallel to a longitudinal axis of the expandable device 100. The connector section length 112 can be the same/uniform or different among different connector sections 110 of a single device 100. The connector section length 112 can be 10 to 450 µm, for example 300 µm.

According to some embodiments, a bridge section length 152 is a longitudinal distance between opposing longitudinal sides of a bridge section 150 (e.g., between a pair of connector sections 110). The bridge section length 152 can be measured as parallel to a longitudinal axis of the expandable device 100. The bridge section length 152 can be the same/uniform or different among different bridge sections 150 of a single device 100. The bridge section length 152 can be 500 to 4500 µm, for example 1,100 µm.

According to some embodiments, some or all of the bridge members 160 and/or some or all of the connector struts 120 can comprise a radiopaque marker. The radiopaque marker can be disposed on a substantially straight section of a bridge member 160 and/or a connector strut 120, so the radiopaque marker is predominantly not subject to bending or flexing. For example, the radiopaque marker(s) can be disposed a distance away from an apex 130. The radiopaque marker(s) can be formed on the bridge members 160 and/or the connector struts 120 by a process that is the same or different than a process used to form the bridge members 160 and/or the connector struts 120, which are discussed further herein.

According to some embodiments, the expandable device 100 can provide a porosity that is the range of 5%-95%. The cells of the expandable device 100 can provide a pore size that is between 5 and 450 µm. A pore size can be measured via a maximum inscribed circle technique.

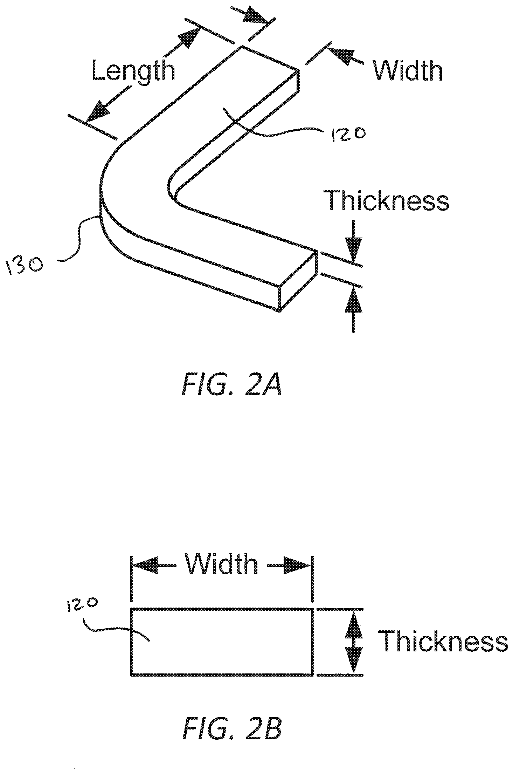

FIG. 2A depicts a perspective view of a connector strut 120 according to some embodiments of the subject technology. FIG. 2B depicts a cross-sectional view of the connector strut 120 according to one aspect of the subject technology. As shown, the connector strut 120 has a length, a width, and a thickness. The thickness can be measured as a dimension that is orthogonal to a central axis when the expandable device 100 is considered in a tubular shape or as a dimension that is orthogonal to a plane of the expandable device 100 when represented as laid-flat. The length can be measured as a distance extending between ends of a strut, where the ends connect to another structure. The width can be measured as the distance that is generally orthogonal to the length and thickness. The width and length of a strut can contribute to a surface coverage and porosity of the expandable device 100. According to some embodiments, the connector strut 120 can have a square cross-section. According to some embodiments, the bridge member 160 can have a similar square cross-section. However, the connector strut 120 and/or the bridge member 160 may have other suitable cross-sectional shapes, such as rectangular, polygonal, round, ovoid, elliptical, or combinations thereof.

According to some embodiments, a thickness of the connector struts 120 and/or the bridge members 160 can be 5 to 50 µm, for example 50 µm. According to some embodiments, a width of the connector struts 120 and/or the bridge members 160 can be 5 to 50 µm, for example 50 µm.

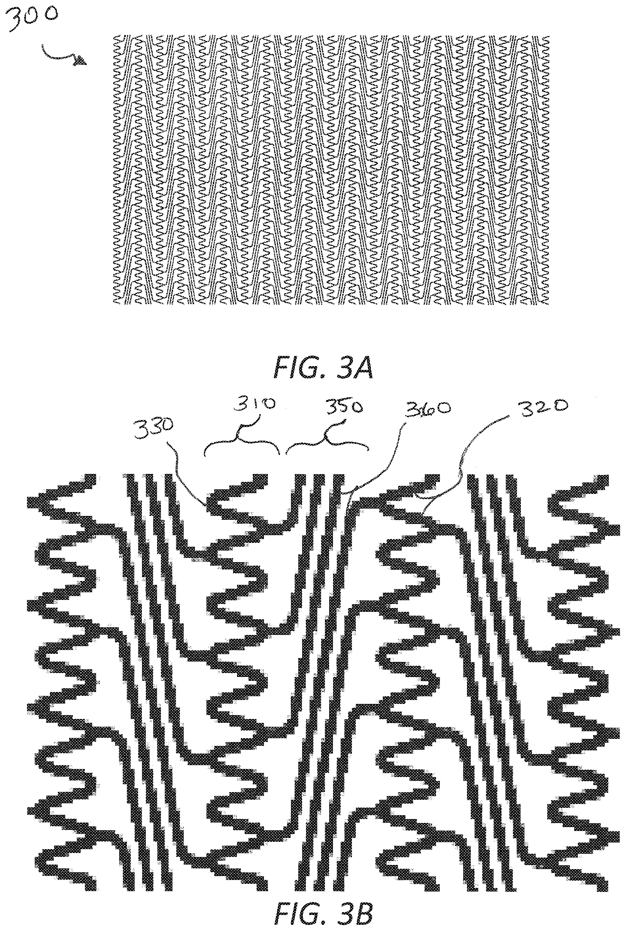

According to some embodiments, for example as shown in FIGS. 3A and 3B, an expandable device can have a number of apices that is greater than a number of bridge members, such that at least some of the apices do not connect directly to a bridge member.

According to some embodiments, for example as shown in FIGS. 3A and 3B, an expandable device 300 can comprise a plurality of connector struts 320 and apices 330 within a plurality of connector sections 310 and a plurality of bridge members 360 within a plurality of bridge sections 350. Features of the expandable device 300 that are identified with reference numerals that differ from the reference numerals for the expandable device 100 by a multiple of 100 can have the same aspects as the corresponding features in the expandable device 100, unless noted otherwise.

According to some embodiments, for example as shown in FIGS. 3A and 3B, at least some of the apices 330 do not connect directly to a bridge member 360. For example, a number of apices 330 or connector struts 320 can be greater than a number of bridge members 360. Accordingly, at least some of the connector struts 320 terminate at an apex 330 that does not connect to a bridge member 360. For example, a given connector section 310 can form a number of apices 330 that face an adjacent bridge section 350, and the bridge section 350 can comprise fewer (for example, one-half or one-third) bridges than such adjacent, facing apices. Accordingly, every other (or every third, fourth, fifth, etc.) adjacent, facing apex 330 can be connected to a bridge of the adjacent bridge section 350, and the remaining apices can be unconnected to a bridge.

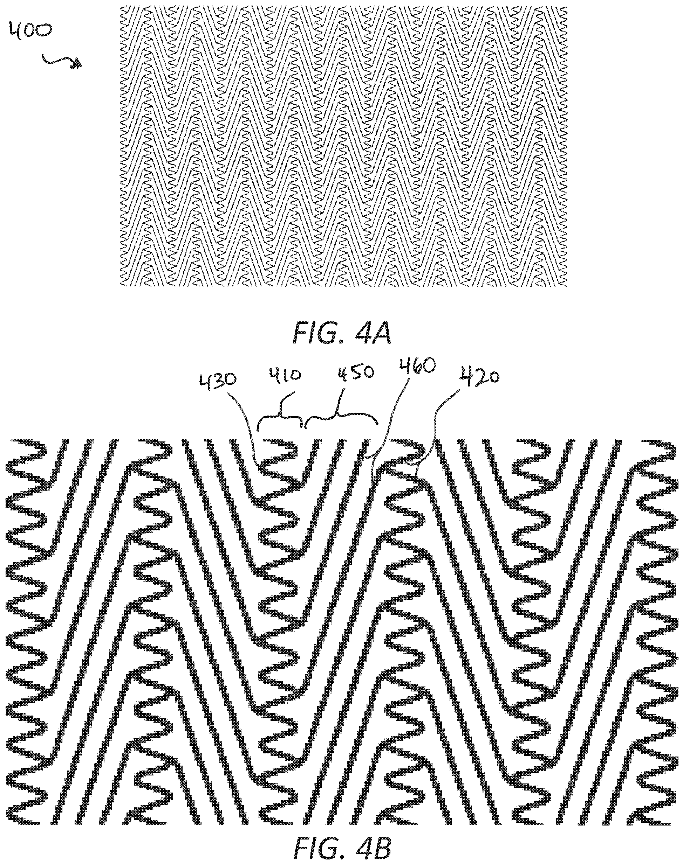

According to some embodiments, for example as shown in FIGS. 4A and 4B, an expandable device 400 can comprise a plurality of connector struts 420 and apices 430 within a plurality of connector sections 410 and a plurality of bridge members 460 within a plurality of bridge sections 450. Features of the expandable device 400 that are identified with reference numerals that differ from the reference numerals for the expandable device 100 by a multiple of 100 can have the same aspects as the corresponding features in the expandable device 100, unless noted otherwise.

According to some embodiments, for example as shown in FIGS. 4A and 4B, at least some of the apices 430 do not connect directly to a bridge member 460. For example, a number of apices 430 or connector struts 420 can be greater than a number of bridge members 460. Accordingly, at least some of the connector struts 420 terminate at an apex 430 that does not connect to a bridge member 460. For example, a given connector section 410 can form a number of apices 430 that face an adjacent bridge section 450, and the bridge section 450 can comprise fewer (for example, one-half or one-third) bridges than such adjacent, facing apices. Accordingly, every other (or every third, fourth, fifth, etc.) adjacent, facing apex 430 can be connected to a bridge of the adjacent bridge section 450, and the remaining apices can be unconnected to a bridge.

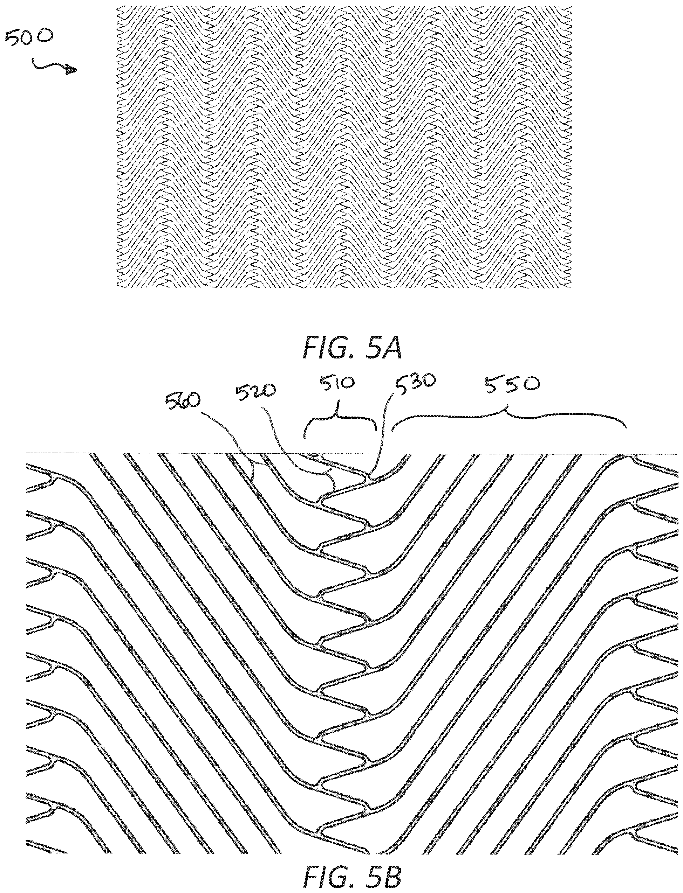

According to some embodiments, for example as shown in FIGS. 5A and 5B, bridge members of an expandable device can connect to connector struts at a location other than at an apex where two connector struts are coupled together.

According to some embodiments, for example as shown in FIGS. 5A and 5B, an expandable device 500 can comprise a plurality of connector struts 520 and apices 530 within a plurality of connector sections 510 and a plurality of bridge members 560 within a plurality of bridge sections 550. Features of the expandable device 500 that are identified with reference numerals that differ from the reference numerals for the expandable device 100 by a multiple of 100 can have the same aspects as the corresponding features in the expandable device 100, unless noted otherwise.

According to some embodiments, for example as shown in FIGS. 5A and 5B, some or all of the bridge members 560 connect to a connector section 510 at a location that is not, or is slightly offset from a centerline of, an apex 530 of two connector struts 520. Instead, some or all of the bridge members 560 connect more closely to one connector strut 520 than to the other connector strut 520 with which it forms an apex 530. In this configuration, the connection to the bridge member 560 is less likely to interfere with the flexing of the apex 530.

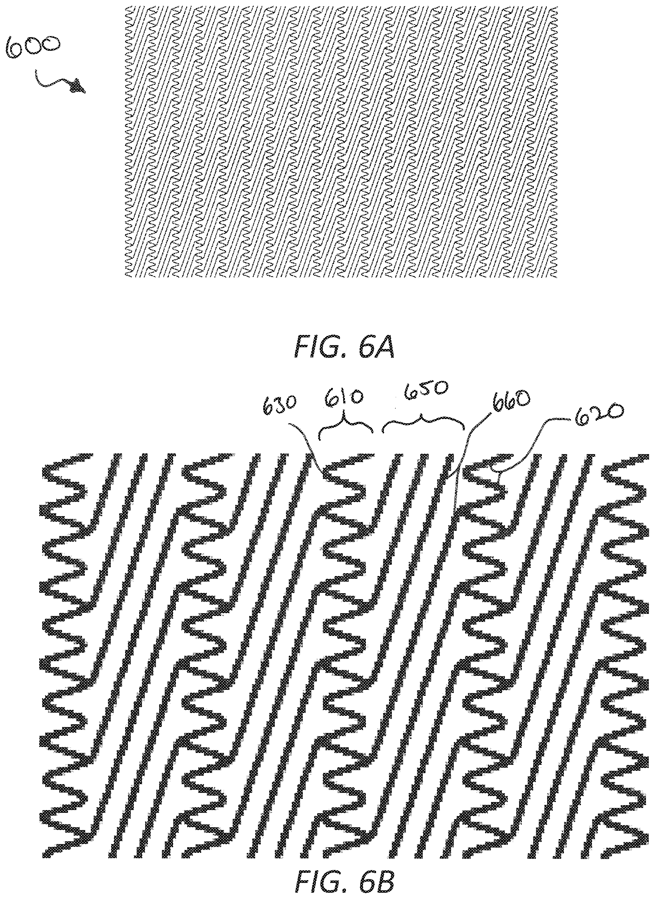

According to some embodiments, for example as shown in FIGS. 6A and 6B, each and every bridge member of an expandable device can extend in the same helical winding direction.

According to some embodiments, for example as shown in FIGS. 6A and 6B, an expandable device 600 can comprise a plurality of connector struts 620 and apices 630 within a plurality of connector sections 610 and a plurality of bridge members 660 within a plurality of bridge sections 650. Features of the expandable device 600 that are identified with reference numerals that differ from the reference numerals for the expandable device 100 by a multiple of 100 can have the same aspects as the corresponding features in the expandable device 100, unless noted otherwise.

According to some embodiments, for example as shown in FIGS. 6A and 6B, a helical winding direction of the bridge members 660 of one bridge section 650 can be the same as a helical winding direction of the bridge members 660 of a different bridge section 650. For example, the helical winding direction of all bridge members 660 of all bridge sections 650 can be dextrorotatory or levorotatory. When the expandable device 600 is extended longitudinally, the bridge members 660 of the bridge sections 650 can straighten relative to the longitudinal axis, causing the connector sections 610 to rotate about the axis in the same direction. This allows the extreme ends of the expandable device 600 to rotate relative to each other in the same way throughout the expansion of the expandable device 600.

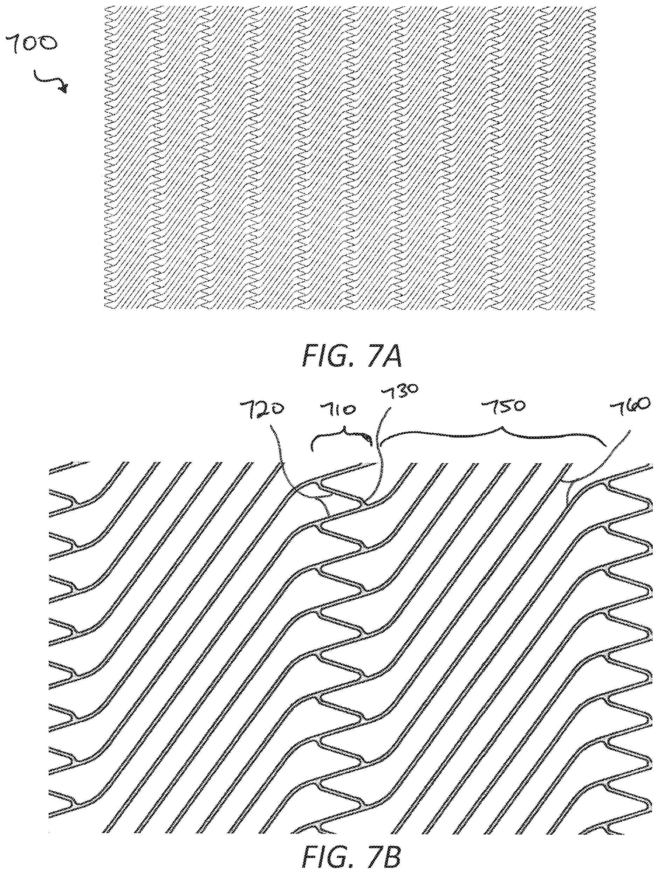

According to some embodiments, for example as shown in FIGS. 7 A and 7B, an expandable device can incorporate (1) the connection of struts as described with respect to the expandable device 500 and (2) the helical winding direction as described with respect to the expandable device 600.

According to some embodiments, for example as shown in FIGS. 7 A and 7B, an expandable device 700 can comprise a plurality of connector struts 720 and apices 730 within a plurality of connector sections 710 and a plurality of bridge members 760 within a plurality of bridge sections 750. Features of the expandable device 700 that are identified with reference numerals that differ from the reference numerals for the expandable device 100 by a multiple of 100 can have the same aspects as the corresponding features in the expandable device 100, unless noted otherwise.

According to some embodiments, for example as shown in FIGS. 7 A and 7B, a helical winding direction of the bridge members 760 of one bridge section 750 can be the same as a helical winding direction of the bridge members 760 of a different bridge section 750. According to some embodiments, some or all of the bridge members 760 connect to a connector section 710 at a location that is not at, or is slightly offset from, a centerline of an apex 730 of two connector struts 720. Instead, some or all of the bridge members 760 connect more closely to one connector strut 720 than to the other connector strut 720 with which it forms an apex 730. According to some embodiments, at least some of the apices 730 do not connect directly to a bridge member 760.

According to some embodiments, for example as shown in FIGS. 8A and 8B, an expandable device can incorporate (1) the unconnected apices described with respect to the expandable device 300 and (2) the connection of struts as described with respect to the expandable device 500.

According to some embodiments, for example as shown in FIGS. 8A and 8B, an expandable device 800 can comprise a plurality of connector struts 820 and apices 830 within a plurality of connector sections 810 and a plurality of bridge members 860 within a plurality of bridge sections 850. Features of the expandable device 800 that are identified with reference numerals that differ from the reference numerals for the expandable device 100 by a multiple of 100 can have the same aspects as the corresponding features in the expandable device 100, unless noted otherwise.

According to some embodiments, for example as shown in FIGS. 8A and 8B, a helical winding direction of the bridge members 860 of one bridge section 850 can be different than a helical winding direction of the bridge members 860 of a different bridge section 850. According to some embodiments, some or all of the bridge members 860 connect to a connector section 810 at a location that is not at, or is slightly offset from, a centerline of an apex 830 of two connector struts 820. Instead, some or all of the bridge members 860 connect more closely to one connector strut 820 than to the other connector strut 820 with which it forms an apex 830. According to some embodiments, at least some of the apices 830 do not connect directly to a bridge member 860.

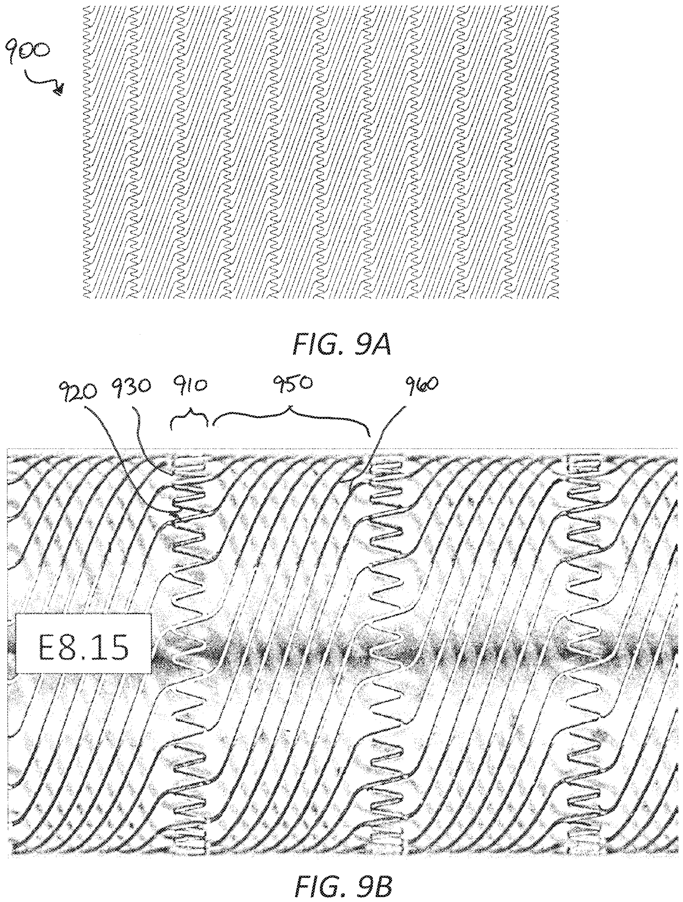

According to some embodiments, for example as shown in FIGS. 9A and 9B, an expandable device can incorporate (1) the unconnected apices described with respect to the expandable device 300, (2) the connection of struts as described with respect to the expandable device 500, and (3) the helical winding direction as described with respect to the expandable device 600.

According to some embodiments, for example as shown in FIGS. 9A and 9B, an expandable device 900 can comprise a plurality of connector struts 920 and apices 930 within a plurality of connector sections 910 and a plurality of bridge members 960 within a plurality of bridge sections 950. Features of the expandable device 900 that are identified with reference numerals that differ from the reference numerals for the expandable device 100 by a multiple of 100 can have the same aspects as the corresponding features in the expandable device 100, unless noted otherwise.

According to some embodiments, for example as shown in FIGS. 9A and 9B, a helical winding direction of the bridge members 960 of one bridge section 950 can be the same a helical winding direction of the bridge members 960 of a different bridge section 950. According to some embodiments, some or all of the bridge members 960 connect to a connector section 910 at a location that is not at, or is slightly offset from, a centerline of an apex 930 of two connector struts 920. Instead, some or all of the bridge members 960 connect more closely to one connector strut 920 than to the other connector strut 920 with which it forms an apex 930. According to some embodiments, at least some of the apices 930 do not connect directly to a bridge member 960.

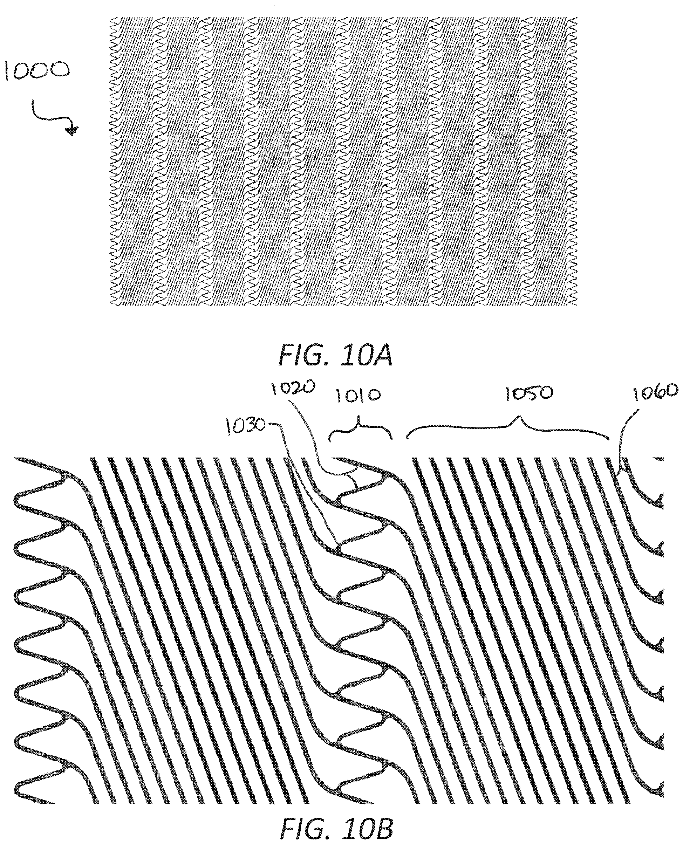

According to some embodiments, for example as shown in FIGS. 10A and 10B, an expandable device can incorporate (1) the connection of struts as described with respect to the expandable device 500 and (2) the helical winding direction as described with respect to the expandable device 600.

According to some embodiments, for example as shown in FIGS. 10A and 10B, an expandable device 1000 can comprise a plurality of connector struts 1020 and apices 1030 within a plurality of connector sections 1010 and a plurality of bridge members 1060 within a plurality of bridge sections 1050. Features of the expandable device 1000 that are identified with reference numerals that differ from the reference numerals for the expandable device 100 by a multiple of 100 can have the same aspects as the corresponding features in the expandable device 100, unless noted otherwise.

According to some embodiments, for example as shown in FIGS. 10A and 10B, a helical winding direction of the bridge members 1060 of one bridge section 1050 can be the same as a helical winding direction of the bridge members 1060 of a different bridge section 1050. According to some embodiments, some or all of the bridge members 1060 connect to a connector section 1010 at a location that is not at, or is slightly offset from, a centerline of an apex 1030 of two connector struts 1020.

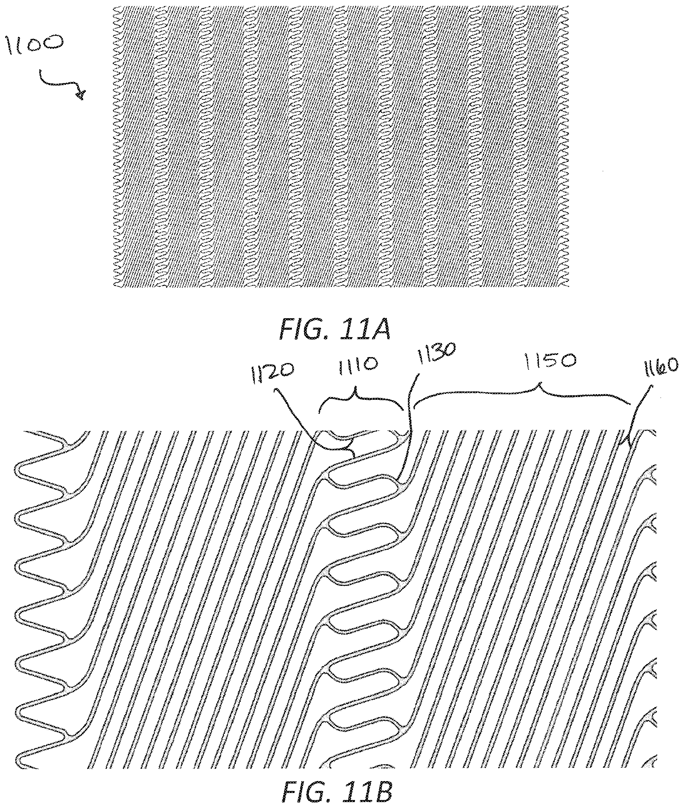

According to some embodiments, for example as shown in FIGS. 11A and 1 1B, an expandable device can comprise some connector struts that are curved between apices, wherein the connector struts are parallel to each in a helical winding direction. For example, at least a portion of each of the connector struts 1120 that are joined at a given apex 1130 (or at some or all apices 1130 of one, some or all connector sections 1110) are parallel to each other. In a given connector section every other connector strut 1120 can have a curved section near each end, and a straight midsection between the two curved sections. Such a configuration can shift some of the strain of device compression or expansion from the apices 1130 to the curved sections to avoid over-straining or distorting the apices, and/or to allow a greater degree of device compression or expansion.

According to some embodiments, for example as shown in FIGS. 11A and 11B, an expandable device 1100 can comprise a plurality of connector struts 1120 and apices 1130 within a plurality of connector sections 1110 and a plurality of bridge members 1160 within a plurality of bridge sections 1150. Features of the expandable device 1100 that are identified with reference numerals that differ from the reference numerals for the expandable device 100 by a multiple of 100 can have the same aspects as the corresponding features in the expandable device 100, unless noted otherwise.

According to some embodiments, for example as shown in FIGS. 11A and 11B, a helical winding direction of the bridge members 1160 of one bridge section 1150 can be the same as a helical winding direction of the bridge members 1160 of a different bridge section 1150. According to some embodiments, some or all of the bridge members 1160 connect to a connector section 1110 at a location that is not at, or is slightly offset from, a centerline of an apex 1130 of two connector struts 1120. Optionally, the connector section 1110 located at one or both longitudinal terminal ends of the device 1100 can comprise a V-strut band such as the connector section 110 of FIGS. 1A-1B.

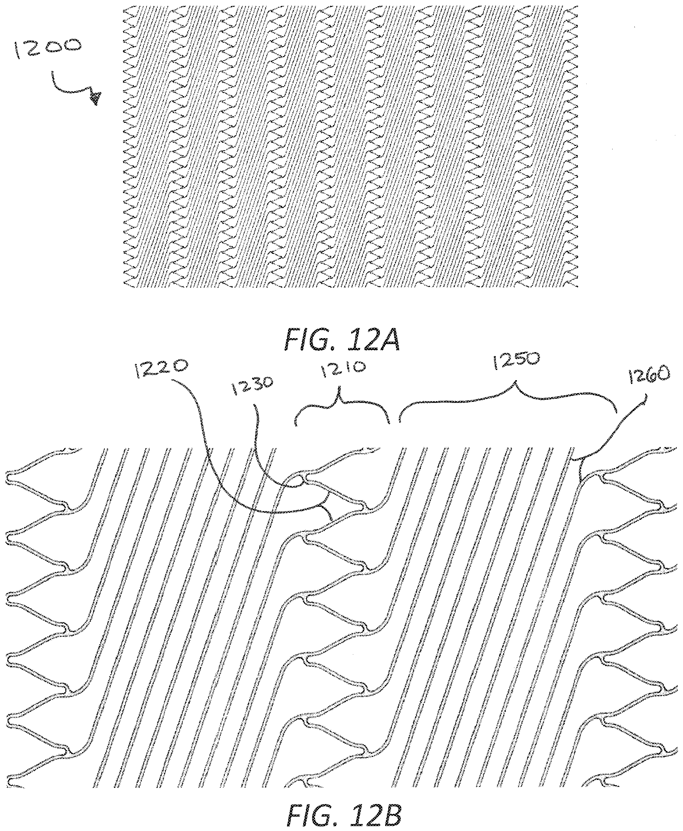

According to some embodiments, for example as shown in FIGS. 12A and 12B, an expandable device can have connector struts that are coupled together at an apices that have a curve that is configured to reduce a bend radius at each apex.

According to some embodiments, for example as shown in FIGS. 12A and 12B, the apices 1230 can have a shape that is configured to reduce a bend radius at each apex 1230 while preserving the size and relative orientation of the connector struts 1220. For example, some or all of the connector struts 1220 can be curved so that as a pair of struts 1220 approach an apex 1230, the struts 1220 turn away from each other forming generally parallel terminal portions that extend to the apex 1230. This allows for a smaller bend radius at the apex 1230 while preserving the size and angle of the V formed by the struts 1220. During compression or expansion of the device 1200, this tends to concentrate bending of each V at the apex 1230 rather than along the struts 1220 which can cause undesirable distortion or out-of-plane movement of the struts 1220. The inside edge of the apex 1230 can be made semicircular. When the expandable device 1200 is compressed radially, the connector struts 1220 move closer to each other by bending about the apices 1230. The connector struts 1220 at terminal ends of the expandable device 1200 can have a different shape (e.g., the shape of the connector struts 120 of the expandable device 100).

According to some embodiments, for example as shown in FIGS. 12A and 12B, an expandable device 1200 can comprise a plurality of connector struts 1220 and apices 1230 within a plurality of connector sections 1210 and a plurality of bridge members 1260 within a plurality of bridge sections 1250. Features of the expandable device 1200 that are identified with reference numerals that differ from the reference numerals for the expandable device 100 by a multiple of 100 can have the same aspects as the corresponding features in the expandable device 100, unless noted otherwise.

According to some embodiments, for example as shown in FIGS. 12A and 12B, a helical winding direction of the bridge members 1260 of one bridge section 1250 can be the same as a helical winding direction of the bridge members 1260 of a different bridge section 1250. According to some embodiments, some or all of the bridge members 1260 connect to a connector section 1210 at a location that is not an apex 1230 of two connector struts 1220.

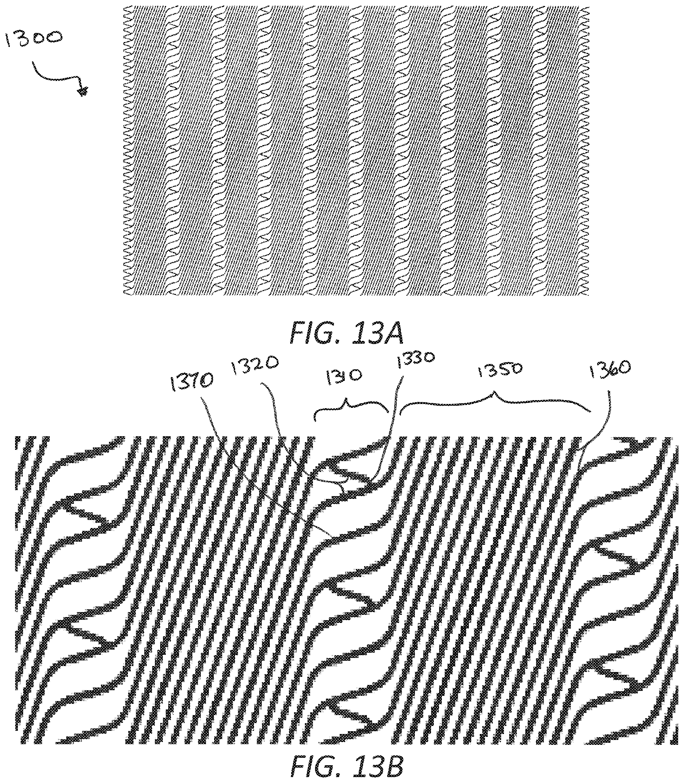

According to some embodiments, for example as shown in FIGS. 13A and 13B, an expandable device can have some bridge members that terminate at a pass-through strut 1370 that extends through a connector section 1310 without contacting or being connected to any connector struts or apex.

According to some embodiments, for example as shown in FIGS. 13A and 13B, an expandable device 1300 can comprise a plurality of connector struts 1320 and apices 1330 within a plurality of connector sections 1310 and a plurality of bridge members 1360 within a plurality of bridge sections 1350. Features of the expandable device 1300 that are identified with reference numerals that differ from the reference numerals for the expandable device 100 by a multiple of 100 can have the same aspects as the corresponding features in the expandable device 100, unless noted otherwise.

According to some embodiments, for example as shown in FIGS. 13A and 13B, a helical winding direction of the bridge members 1360 of one bridge section 1350 can be the same as a helical winding direction of the bridge members 1360 of a different bridge section 1350. According to some embodiments, some or all of the bridge members 1360 connect to a connector section 1310 at a location that is not an apex 1330 of two connector struts 1320.

According to some embodiments, for example as shown in FIGS. 13A and 13B, some of the bridge members 1360 can terminate at a pass-through strut 1370 that extends through the connector section 1310 without contacting or being connected to any connector struts 1320 or apex 1330. The pass-through struts 1370 can extend from one bridge section 1350 to another bridge section 1350 on an opposing side of the connector section 1310. Only some (e.g., 1/3) of the bridge members 1360 that connect to a given connector section 1310 connect to a pass-through strut 1370 at that connector section 1310. The remainder of the bridge members 1360 can connect to a connector strut 1320 or apex 1330 at that connector section 1310. Each bridge member 1360 that connects to a pass-through strut 1370 on one terminal end of the bridge member 1360 can connect to a connector strut 1320 and/or an apex 1330 at the opposite terminal end of the bridge member 1360. Optionally, the connector section 1310 located at one or both longitudinal terminal ends of the device 1300 can comprise a V-strut band such as the connector section 110 of FIGS. 1A to 1B.

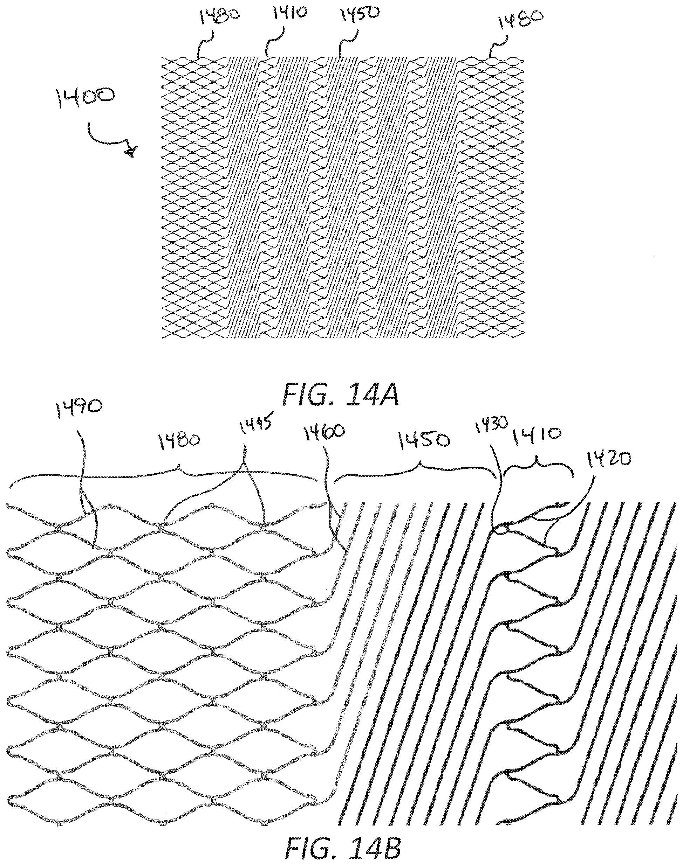

According to some embodiments, for example as shown in FIGS. 14A and 14B, an expandable device can have an end section at one or both of its longitudinally terminal ends to provide securement of the expandable device within a body vessel.

According to some embodiments, for example as shown in FIGS. 14A and 14B, an expandable device 1400 can comprise a plurality of connector struts 1420 and apices 1430 within a plurality of connector sections 1410 and a plurality of bridge members 1460 within a plurality of bridge sections 1450. Features of the expandable device 1400 that are identified with reference numerals that differ from the reference numerals for the expandable device 100 by a multiple of 100 can have the same aspects as the corresponding features in the expandable device 140, unless noted otherwise.

According to some embodiments, for example as shown in FIGS. 14A and 14B a helical winding direction of the bridge members 1460 of one bridge section 1450 can be the same as a helical winding direction of the bridge members 1460 of a different bridge section 1450. According to some embodiments, some or all of the bridge members 1460 connect to a connector section 1410 at a location that is not an apex 1430 of two connector struts 1420.

According to some embodiments, for example as shown in FIGS. 14A and 14B the expandable device 1400 can comprise an end section 1480 at one or both of its longitudinally terminal ends. The end sections 1480 can be generally stiffer than the bridge sections 1450. Each of the end sections 1480 can comprise end struts 1490. The end struts 1490 can be interconnected at apices 1495. The end struts 1490 can form a series of undulations (e.g., sinusoidal or "S-curves") that extend longitudinally across the some or all of the end section 1480. The end struts 1490 can be connected to each other at or near peaks or troughs thereof. The end struts 1490 can be arranged to form a series of cells that are similar in size and shape. For example, the cells can be approximately diamond shaped. The end struts 1490 can be shorter than the bridge members 1460. For example, the end struts 1490 can be approximately the same length as the connector struts 1420. The end struts 1490 can comprise a series of longitudinally adjacent V-strut bands like that employed as the connector sections 1410. In such a configuration, every other band can be inverted longitudinally and the bands connected apex-to-apex as shown in Fig. 14B.

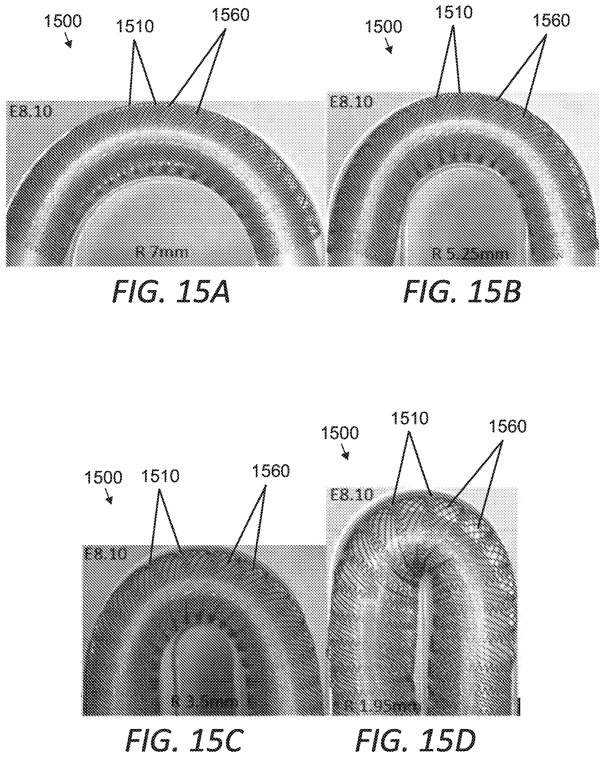

According to some embodiments, as shown in FIGS. 15A-D, when an expandable device 1500 is bent to conform to a body vessel with tortuous curvature, the connector sections 1510 can move closer to each other on the "inside-curving" side of the expandable device 1500 when the bridge members 1560 collapse longitudinally and move closer to each other on that side. The thinness and arrangement of struts provides enhanced longitudinal flexibility and better arching capability. When deployed in a tortuous body vessel, the device of the subject technology will readily bend at a bridge section, thus providing improved wall apposition at a curve. For example, referring to FIGS. 15A-D, the device 1500 is disposed in a body vessel with tortuous curvature. At an apex of a curve in the body vessel, the bridge members 1560 adjacent the apex move toward each other to facilitate contact with an inner surface of the vessel, thereby providing improved wall apposition near the apex. A distance between bridge members 1560 adjacent to the apex of the curve is less than a distance between bridge members 1560 disposed away from the apex. By allowing the bridge members 1560 to move near each other, the bridge members 1560 may better conform to the shape of the curve. This helps avoid issues that may occur in other devices, such as tendencies to ovalize, kink, or fish mouth when placed in a body vessel with tortuous curvature.

An expandable device may be formed, for example, by laser cutting a preformed tube or sheet, by interconnecting components (e.g., by laser welding), by vapor deposition techniques, or combinations thereof. The expandable device can be formed using known flexible materials such as nitinol, stainless steel, cobalt-chromium alloys, Elgiloy, magnesium alloys, tungsten, tantalum, platinum, or combinations thereof.

According to some embodiments, an expandable device can be formed by a photolithography process. A substrate can be provided with a base for supporting the formation of the expandable device. The base (e.g., copper) can be used temporarily as a buffer between the substrate and a primary material used to form the expandable device. After the base is provided on the substrate, the primary material is provided thereon, for example by vapor deposition. The primary material can be provided as a thin film of substantially uniform thickness. Portions of the primary material can be removed to form the structure of the expandable device. For example, a photomask, based on a strut pattern, can be used to selectively expose portions of the primary material to light and etch the primary material into the desired shape for the expandable device. Alternatively or in combination, a chemical agent can be used to remove the portions of the primary material that are not protected by a photoresist. The base can then be eroded to separate the expandable device from the substrate. The expandable device can be further treated to form a desired shape (e.g., tubular) and have the desired heat set and/or shape memory properties.

According to some embodiments, an expandable device can be formed by a laser cutting process. The expandable device may be formed by cutting a pattern of struts on a tube or on a flat sheet and then rolling the flat sheet into a generally tube-like or coiled shape. The expandable device in a generally tube-like or coiled shape can be circumferentially continuous or discontinuous. Where the expandable device is circumferentially discontinuous, portions of the expandable device can overlap in certain states.

As mentioned elsewhere herein, the present disclosure also includes methods of treating a vascular condition, such as an aneurysm, with any of the embodiments of the expandable devices disclosed herein. The expandable device could be deployed across the neck of an aneurysm and its flow-diverting properties employed to impede blood flow between the aneurysm and the parent vessel, cause the blood inside the aneurysm to thrombose, and lead to healing of the aneurysm.

In order to implant any of the expandable devices disclosed herein, the expandable device can be mounted in a delivery system. Generally, the delivery system can comprise an elongate core member that supports or contains the expandable device, and both components can be slidably received in a lumen of a microcatheter or other elongate sheath for delivery to any region to which the distal opening of the microcatheter can be advanced. The core member is employed to advance the expandable device through the microcatheter and out the distal end of the microcatheter so that the expandable device is allowed to self-expand into place in the blood vessel, across an aneurysm or other treatment location. Accordingly, a vascular treatment apparatus can comprise a delivery system, such as any of the delivery systems described herein, and an expandable device, such as any of the expandable devices described herein, mounted in the delivery system.

A treatment procedure can begin with obtaining percutaneous access to the patient's arterial system, typically via a major blood vessel in a leg or arm. A guidewire can be placed through the percutaneous access point and advanced to the treatment location, which can be in an intracranial artery, or any neurovascular artery, peripheral artery or coronary artery. (As configured for neurovascular use, any of the expandable devices disclosed herein can have a diameter of 2-8 mm in the relaxed state or the expanded state; expandable devices used in the peripheral or coronary vasculature can have a diameter of 1-20 mm in the relaxed state or the expanded state.) The microcatheter is then advanced over the guidewire to the treatment location and situated so that a distal open end of the guidewire is adjacent to the treatment location. The guidewire can then be withdrawn from the microcatheter and the core member, together with the expandable device mounted thereon or supported thereby, can be advanced through the microcatheter and out the distal end thereof. The expandable device can then self-expand into apposition with the inner wall of the blood vessel. Where an aneurysm is being treated, the expandable device is placed across the neck of the aneurysm so that a sidewall of the expandable device separates the interior of the aneurysm from the lumen of the parent artery. Once the expandable device has been placed, the core member and microcatheter are removed from the patient. The expandable device sidewall can now perform a flow-diverting function on the aneurysm, thrombosing the blood in the aneurysm and leading to healing of the aneurysm.

The foregoing description is provided to enable a person skilled in the art to practice the various configurations described herein. While the subject technology has been particularly described with reference to the various figures and configurations, it should be understood that these are for illustration purposes only and should not be taken as limiting the scope of the subject technology.

There may be many other ways to implement the subject technology. Various functions and elements described herein may be partitioned differently from those shown without departing from the scope of the subject technology. Various modifications to these configurations will be readily apparent to those skilled in the art, and generic principles defined herein may be applied to other configurations. Thus, many changes and modifications may be made to the subject technology, by one having ordinary skill in the art, without departing from the scope of the subject technology.

A phrase such as "an aspect" does not imply that such aspect is essential to the subject technology or that such aspect applies to all configurations of the subject technology. A disclosure relating to an aspect may apply to all configurations, or one or more configurations. An aspect may provide one or more examples of the disclosure. A phrase such as "an aspect" may refer to one or more aspects and vice versa. A phrase such as "an embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples of the disclosure. A phrase such "an embodiment" may refer to one or more embodiments and vice versa. A phrase such as "a configuration" does not imply that such configuration is essential to the subject technology or that such configuration applies to all configurations of the subject technology. A disclosure relating to a configuration may apply to all configurations, or one or more configurations. A configuration may provide one or more examples of the disclosure. A phrase such as "a configuration" may refer to one or more configurations and vice versa.

It is understood that the specific order or hierarchy of steps in the processes disclosed is an illustration of exemplifying approaches. Based upon design preferences, it is understood that the specific order or hierarchy of steps in the processes may be rearranged. Some of the steps may be performed simultaneously. Various methods are disclosed presenting elements of the various steps in a sample order, and are not meant to be limited to the specific order or hierarchy presented.

Furthermore, to the extent that the term "include," "have," or the like is used herein, such term is intended to be inclusive in a manner similar to the term "comprise" as "comprise" is interpreted when employed as a transitional word in a claim.

A reference to an element in the singular is not intended to mean "one and only one" unless specifically stated, but rather "one or more." The term "some" refers to one or more. All structural and functional equivalents to the elements of the various configurations described throughout this disclosure that are known or later come to be known to those of ordinary skill in the art are intended to be encompassed by the subject technology. Moreover, nothing disclosed herein is intended to be dedicated to the public regardless of whether such disclosure is explicitly recited in the above description.

While certain aspects and embodiments of the subject technology have been described, these have been presented by way of example only, and are not intended to limit the scope of the subject technology.

## Claims

1. An expandable device (1100) comprising:
a plurality of connector sections (1110), each of the connector sections (1110) extending circumferentially about the expandable device (1100) and comprising a plurality of connector struts (1120), the connector struts (1120) including first connector struts and second connector struts, wherein (a) the first connector struts are linear, (b) every other connector strut (1120) is a second connector strut, (c) each of the second connector struts comprises a curved section near each end and a straight midsection between the two curved sections, the curved sections of each second connector strut curving in opposite directions, and (d) each of the first connector struts is coupled to one of the second connector struts at an apex (1130); and
a plurality of bridge sections (1150), each of the bridge sections (1150) attached to and extending between two of the connector sections (1110) and comprising a plurality of parallel, non-branching, helical bridge members, wherein at least some of the bridge members (1160) directly connect to one of the apices (1130), wherein, for each pair of first connector struts and second connector struts connected by one of the apices (1130), at least the straight midsection of the second connector strut is parallel to at least a portion of the first connector strut.

2. The expandable device (1100) of claim 1, wherein at least some of the bridge members individually connect to a corresponding connector section (1110) at a location that is offset from a centerline of the one of the apices (1130) to which the respective bridge member (1160) connects.

3. The expandable device (1100) of claim 1, wherein a first one of the bridge sections (1150) comprises first bridge members winding in a first helical direction about an axis of the expandable device (1100) and a second one of the bridge sections (1150) comprises second bridge members winding in a second helical direction about the axis of the expandable device, the first helical direction being the same as the second helical direction.

4. The expandable device (1100) of claim 1, wherein each of the apices (1130) is coupled to one of the bridge members (1160).

5. The expandable device (1100) of claim 1, wherein the connector struts (1120) are parallel to each other in a helical winding direction.

6. The expandable device (1100) of claim 1, further comprising anchor sections at longitudinal ends of the expandable device (1100), each of the anchor sections comprising a plurality of closed cells.

7. The expandable device (1100) of claim 1, wherein the expandable device is a mesh.

8. The expandable device (1100) of claim 7, wherein the mesh is a laser cut sheet.

9. The expandable device (1100) of claim 7, wherein the mesh is non-braided.

10. The expandable device (1100) of claim 1, wherein the expandable device (1100) is self-expanding.

## Patentansprüche

1. Erweiterbare Vorrichtung (1100), umfassend:
eine Vielzahl von Verbindungsabschnitten (1110), wobei sich jeder der Verbindungsabschnitte (1110) kreisförmig um die erweiterbare Vorrichtung (1100) erstreckt und eine Vielzahl von Verbindungsstreben (1120) umfasst, wobei die Verbindungsstreben (1120) erste Verbindungsstreben und zweite Verbindungsstreben einschließen, wobei (a) die ersten Verbindungsstreben linear sind, (b) jede zweite Verbindungsstrebe (1120) eine zweite Verbindungsstrebe ist, (c) jede der zweiten Verbindungsstreben einen gekrümmten Abschnitt in der Nähe jedes Endes und einen geraden Mittelabschnitt zwischen den zwei gekrümmten Abschnitten umfasst, wobei die gekrümmten Abschnitte jeder zweiten Verbindungsstrebe in entgegengesetzte Richtungen gekrümmt sind, und (d) jede der ersten Verbindungsstreben an einem Scheitelpunkt (1130) mit einer der zweiten Verbindungsstreben gekoppelt ist; und
eine Vielzahl von Brückenabschnitten (1150), wobei jeder der Brückenabschnitte (1150) an zwei der Verbindungsabschnitte (1110) angebracht ist und sich zwischen diesen erstreckt und eine Vielzahl von parallelen, nichtverzweigten, spiralförmigen Brückenelementen umfasst, wobei mindestens einige der Brückenelemente (1160) direkt mit einem der Scheitelpunkte (1130) verbunden sind, wobei für jedes Paar aus ersten Verbindungsstreben und zweiten Verbindungsstreben, die durch einen der Scheitelpunkte (1130) verbunden sind, zumindest der gerade Mittelabschnitt der zweiten Verbindungsstrebe parallel zu mindestens einem Teil der ersten Verbindungsstrebe ist.

2. Erweiterbare Vorrichtung (1100) nach Anspruch 1, wobei mindestens einige der Brückenelemente einzeln mit einem entsprechenden Verbindungsabschnitt (1110) an einer Stelle verbunden sind, die von einer Mittellinie des einen der Scheitelpunkte (1130), mit dem das jeweilige Brückenelement (1160) verbunden ist, versetzt ist.

3. Erweiterbare Vorrichtung (1100) nach Anspruch 1, wobei ein erster der Brückenabschnitte (1150) erste Brückenelemente umfasst, die sich in einer ersten Spiralrichtung um eine Achse der erweiterbaren Vorrichtung (1100) winden, und ein zweiter der Brückenabschnitte (1150) zweite Brückenelemente umfasst, die sich in einer zweiten Spiralrichtung um die Achse der erweiterbaren Vorrichtung winden, wobei die erste Spiralrichtung dieselbe ist wie die zweite Spiralrichtung.

4. Erweiterbare Vorrichtung (1100) nach Anspruch 1, wobei jeder der Scheitelpunkte (1130) mit einem der Brückenelemente (1160) gekoppelt ist.

5. Erweiterbare Vorrichtung (1100) nach Anspruch 1, wobei die Verbindungsstreben (1120) in einer spiralförmigen Windungsrichtung parallel zueinander verlaufen.

6. Erweiterbare Vorrichtung (1100) nach Anspruch 1, die ferner Ankerabschnitte an Längsenden der erweiterbaren Vorrichtung (1100) umfasst, wobei jeder der Ankerabschnitte eine Vielzahl geschlossener Zellen umfasst.

7. Erweiterbare Vorrichtung (1100) nach Anspruch 1, wobei die erweiterbare Vorrichtung ein Netz ist.

8. Erweiterbare Vorrichtung (1100) nach Anspruch 7, wobei das Netz eine mit Laser geschnittene Platte ist.

9. Erweiterbare Vorrichtung (1100) nach Anspruch 7, wobei das Netz nicht geflochten ist.

10. Erweiterbare Vorrichtung (1100) nach Anspruch 1, wobei die erweiterbare Vorrichtung (1100) selbsterweiternd ist.

## Revendications

1. Dispositif expansible (1100), comprenant :
une pluralité de sections de connecteur (1110), chacune des sections de connecteur (1110) s'étendant circonférentiellement autour du dispositif expansible (1100) et comprenant une pluralité d'entretoises de connecteur (1120), les entretoises de connecteur (1120) comportant des premières entretoises de connecteur et des secondes entretoises de connecteur, dans lequel (a) les premières entretoises de connecteur sont linéaires, (b) toute autre entretoise de connecteur (1120) est une seconde entretoise de connecteur, (c) chacune des secondes entretoises de connecteur comprend une section incurvée près de chaque extrémité et une section médiane droite entre les deux sections incurvées, les sections incurvées de chaque seconde entretoise de connecteur s'incurvant dans des directions opposées, et (d) chacune des premières entretoises de connecteur est accouplée à l'une des secondes entretoises de connecteur au niveau d'un sommet (1130) ; et
une pluralité de sections de pont (1150), chacune des sections de pont (1150) étant fixée à deux des sections de connecteur (1110) et s'étendant entre elles, et comprenant une pluralité d'éléments de pont parallèles, non ramifiés, hélicoïdaux, dans lequel au moins certains des éléments de pont (1160) se relient directement à l'un des sommets (1130), dans lequel, pour chaque paire de premières entretoises de connecteur et de secondes entretoises de connecteur reliées par l'un des sommets (1130), au moins la section médiane droite de la seconde entretoise de connecteur est parallèle à au moins une partie de la première entretoise de connecteur.

2. Dispositif expansible (1100) selon la revendication 1, dans lequel au moins certains des éléments de pont se relient individuellement à une section de connecteur correspondante (1110) au niveau d'un endroit qui est décalé par rapport à une ligne centrale de l'un parmi les sommets (1130) auquel l'élément de pont respectif (1160) se relie.

3. Dispositif expansible (1100) selon la revendication 1, dans lequel l'une parmi les premières sections de pont (1150) comprend des premiers éléments de pont s'enroulant dans une première direction hélicoïdale autour d'un axe du dispositif expansible (1100) et l'une parmi les secondes sections de pont (1150) comprend des seconds éléments de pont s'enroulant dans une seconde direction hélicoïdale autour de l'axe du dispositif expansible, la première direction hélicoïdale étant la même que la seconde direction hélicoïdale.

4. Dispositif expansible (1100) selon la revendication 1, dans lequel chacun des sommets (1130) est accouplé à l'un des éléments de pont (1160).

5. Dispositif expansible (1100) selon la revendication 1, dans lequel les entretoises de connecteur (1120) sont parallèles l'une à l'autre dans une direction d'enroulement hélicoïdale.

6. Dispositif expansible (1100) selon la revendication 1, comprenant en outre des sections d'ancrage au niveau des extrémités longitudinales du dispositif expansible (1100), chacune des sections d'ancrage comprenant une pluralité de cellules fermées.

7. Dispositif expansible (1100) selon la revendication 1, dans lequel le dispositif expansible est une maille.

8. Dispositif expansible (1100) selon la revendication 7, dans lequel la maille est une feuille découpée au laser.

9. Dispositif expansible (1100) selon la revendication 7, dans lequel la maille est non tressée.

10. Dispositif expansible (1100) selon la revendication 1, dans lequel le dispositif expansible (1100) est auto-expansif.
